(19) Europäisches Patentamt / European Patent Office / Office européen des brevets

(11) **EP 4 168 780 B1**

(12) **FASCICULE DE BREVET EUROPEEN**

(45) Date de publication et mention
de la délivrance du brevet:
**06.08.2025 Bulletin 2025/32**

(51) Classification Internationale des Brevets (IPC):
**G01N 21/83** *(2006.01)* **G01N 21/51** *(2006.01)*
**G01N 21/59** *(2006.01)* **G01N 33/28** *(2006.01)*

(21) Numéro de dépôt: **21735252.5**

(52) Classification Coopérative des Brevets (CPC):
**G01N 21/51; G01N 21/59; G01N 21/83;
G01N 33/2823;** G01N 2201/1241

(22) Date de dépôt: **21.06.2021**

(86) Numéro de dépôt international:
**PCT/EP2021/066878**

(87) Numéro de publication internationale:
**WO 2021/259876 (30.12.2021 Gazette 2021/52)**

(54) **DISPOSITIF ET PROCÉDÉ DE DÉTECTION DU SEUIL DE FLOCULATION D'UN MILIEU COLLOÏDAL, NOTAMMENT D'UN MILIEU COMPRENANT DES ASPHALTÈNES, PAR ADDITION DE SOLVANT ALIPHATIQUE**

VORRICHTUNG UND VERFAHREN ZUR BESTIMMUNG DER FLOCKUNGSSCHWELLE EINES KOLLOIDALEN MEDIUMS, INSBESONDERE EINES ASPHALTEN-HALTIGEN MEDIUMS, DURCH ZUGABE EINES ALIPHATISCHEN LÖSUNGSMITTELS

DEVICE AND METHOD FOR DETECTING THE FLOCCULATION THRESHOLD OF A COLLOIDAL MEDIUM, IN PARTICULAR OF A MEDIUM COMPRISING ASPHALTENES, BY ADDING AN ALIPHATIC SOLVENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.06.2020 EP 20305688**

(43) Date de publication de la demande:
**26.04.2023 Bulletin 2023/17**

(73) Titulaire: **TOTALENERGIES ONETECH**
**92400 Courbevoie (FR)**

(72) Inventeurs:
• **AL FARRA, Ahmad**
**76600 LE HAVRE (FR)**
• **OLIVIER, Jérôme**
**76620 LE HAVRE (FR)**
• **LEPINAY, Martial**
**14790 MOUEN (FR)**
• **CHRISTIEN, Jean**
**14320 SAINT MARTIN DE FONTENAY (FR)**

(74) Mandataire: **Fédit-Loriot**
**22, rue du Général Foy**
**75008 Paris (FR)**

(56) Documents cités:
**WO-A2-2005/003754 FR-A1- 2 566 909
US-B2- 10 281 454 US-B2- 10 422 782**

• **ANDERSEN S I: "Flocculation onset titration of petroleum asphaltenes", ENERGY & FUELS, AMERICAN CHEMICAL SOCIETY, WASHINGTON, DC, US, vol. 13, no. 2, 20 February 1999 (1999-02-20), pages 315 - 322, XP002308505, ISSN: 0887-0624, DOI: 10.1021/EF980211D**

## Description

DOMAINE TECHNIQUE

L'invention a pour objet un dispositif de mesure du seuil de floculation d'un milieu colloïdal par addition de solvant aliphatique et un procédé de mesure du seuil de floculation d'un milieu colloïdal, notamment d'un milieu colloïdal contenant des asphaltènes, par addition de solvant aliphatique mis en œuvre par ledit dispositif de mesure.

ETAT DE LA TECHNIQUE

Les produits pétroliers, et notamment les fiouls ou les résidus de distillation du pétrole, généralement appelés "Produits noirs" dans la profession, sont des systèmes colloïdaux constitués d'asphaltènes - c'est à dire de molécules lourdes très aromatiques possédant des chaînes latérales paraffiniques - qui sont dispersés (ou encore appelés "peptisés") sous forme de micelles dans une phase huileuse. Ces systèmes colloïdaux peuvent être déstabilisés plus ou moins facilement, par exemple par craquage thermique ou par dilution. Ainsi, dans une raffinerie, le procédé de conversion, appelé viscoréduction, peut conduire à une précipitation des asphaltènes sous l'effet des températures élevées du procédé (généralement supérieures à 400°C). De même, la constitution de mélanges contenant de tels systèmes colloïdaux peut générer une précipitation de ces asphaltènes par floculation, en particulier si l'environnement de dilution est du type paraffinique.

Il est donc nécessaire de connaître ou d'estimer les caractéristiques de ces asphaltènes dans les produits noirs, tel un produit pétrolier ou un mélange de produits hydrocarbonés, afin d'évaluer sa stabilité intrinsèque, ainsi que sa réserve de stabilité associée. En effet, plus la réserve de stabilité sera élevée, moins le produit noir sera sujet à des problèmes de précipitation des asphaltènes, ou de compatibilité par dilution avec d'autres espèces chimiques, notamment des bases paraffiniques.

On notera que les fiouls ou résidus de pétrole sont constitués d'une matrice malténique (résines + paraffines) et d'asphaltènes dispersés sous forme colloïdale. Les asphaltènes qui ont un caractère très aromatique sont insolubles avec les paraffines qui ont un caractère aliphatique. Pour qu'un résidu soit stable, il est nécessaire que les asphaltènes soient maintenus en suspension (ou dispersés ou peptisés) dans la matrice huileuse. La peptisation des asphaltènes est assurée par les résines qui ont à la fois un caractère aromatique et un caractère aliphatique. Lorsqu'un résidu a été déstabilisé, les asphaltènes floculent en s'agglomérant sous forme de grosses particules pouvant générer des bouchages de filtres présents dans les différentes unités de traitement, ou encore des détériorations de la métallurgie, par exemple les tuyauteries par encrassement qui conduit à une perte d'efficacité énergétique et de la capacité des pipes.

La caractéristique appelée valeur S (« S-value »), ou encore stabilité intrinsèque, par exemple d'un produit noir, est définie dans la profession ainsi que dans la norme ASTM D7157-18 (Révision 2018) par l'expression suivante :

S=aromaticité des malténes/aromaticité des asphalténes, soit encore S=So/(1-Sa), dans laquelle,

- So représente le pouvoir du milieu à solubiliser les asphaltènes, c'est à dire le caractère aromatique du milieu. Plus celui-ci sera aromatique, plus le So sera élevé.
- Sa est le caractère aromatique des asphaltènes.
- 1-Sa représente l'aromaticité du milieu nécessaire pour solubiliser les asphaltènes présents.

Si S>1, les asphaltènes sont peptisés et sont donc stables. S-1 représente la réserve de stabilité (plus cette réserve sera élevée, moins le produit noir sera sujet à des problèmes de précipitation ou de compatibilité).

La sévérité d'un choc thermique, tel que celui apporté par une distillation ou une viscoréduction agit directement sur l'aromaticité des asphaltènes puisque le craquage thermique provoque la coupure des chaînes alkylées et la condensation des asphaltènes. Les asphaltènes plus condensés et moins ramifiés (Sa plus faibles) vont avoir besoin d'un solvant plus puissant pour rester dispersés. Ainsi, la connaissance de la valeur de S, reliée à celle du Sa, va permettre de préciser les réglages des conditions de marche de l'unité concernée pour que celle-ci soit opérée sans risque de précipitation des asphaltènes, et par conséquent, pour répondre aux différentes exigences en matière de qualité de l'exploitant.

Par ailleurs, la connaissance des valeurs du pouvoir solvant So et du caractère aromatique des asphaltènes Sa est nécessaire pour optimiser le mélange des différents constituants des fiouls. C'est ainsi que, si on ajoute un fluxant (produit capable d'abaisser la viscosité d'un mélange) de pouvoir solvant faible à un produit noir, par exemple viscoréduit, et présentant des valeurs So élevée et Sa faible, on réduit la valeur du So du mélange, ce qui peut conduire à une déstabilisation du produit noir, et par conséquent à une floculation des asphaltènes, car les valeurs So et Sa résultantes seraient trop faibles pour satisfaire la relation S>1, c'est à dire la condition pour que les dites asphaltènes soient peptisées, donc stables.

De façon habituelle, on détermine au laboratoire les valeurs de S et Sa, puis par calcul So, d'un produit noir par une dilution étagée à l'aide d'un solvant paraffinique dudit produit noir, préalablement mélangé à un solvant aromatique. On note le moment où une floculation intervient. La mesure est répétée pour au moins un autre mélange avec un taux de dilution différent. On obtient ainsi des résultats qui permettent par corrélation linéaire d'obtenir les valeurs recherchées de S et Sa, puis d'en déduire par calcul So.

Expérimentalement, le seuil de floculation dans un mélange donné peut être décelé par l'intermédiaire de plusieurs sondes optiques fonctionnant dans l'infrarouge (IR) ou le proche infra-rouge (NIR).

Par exemple, la technique décrite dans les brevets FR-A-2 596 522 ou US-A-4 628 204, de la société Texaco Belgium SA, permet de mesurer par IR le seuil de floculation d'une solution colloïdale pendant sa dilution. Cette mesure nécessite au préalable le bon choix de la sonde optique de mesure (il y a plusieurs sondes) en fonction de la nature et notamment de la présence des asphaltènes plus ou moins importante dans le produit noir à tester. En cas de mauvais choix de l'opérateur, il est alors nécessaire de procéder au nettoyage de l'appareillage, puis à une nouvelle préparation de l'échantillon pour une nouvelle mesure avec une autre sonde, ce qui induit une perte de temps pouvant être supérieure à une heure de temps opérateur, alors que le temps d'une analyse est d'environ 1H30 à 2H00, notamment si c'est le choix d'une sonde différente qui s'avère judicieux.

Un autre exemple est la méthode développée par la société Shell, en collaboration avec son partenaire néerlandais Zematra, fabricant d'appareils d'analyses. Cette méthode, dans laquelle la détection du seuil de floculation du milieu colloïdal s'effectue à l'aide d'une seule sonde, constituée d'une simple fibre optique entourée de verre, n'est malheureusement pas utilisable pour toute la gamme des produits noirs. En effet, le chauffage systématique de l'échantillon à 150°C, outre les problèmes de sécurité, peut provoquer, pour certains types de produits noirs, des dégradations préjudiciables à la mesure du seuil de floculation. Le temps d'une analyse, quant à lui, est relativement long puisque pouvant être supérieur à 5 heures.

Une autre méthode est également proposée pour mesurer la valeur de S sur les produits noirs avec un appareil "Porla", fabriqué par la société finlandaise FMS (Finnish Measurement Systems Ltd), et commercialisé par la société anglaise Med-Lab. Cet appareil utilise une cellule de mesure à circulation continue de l'échantillon à analyser avec une détection optique du seuil de floculation au moyen d'un prisme fonctionnant en réflexion totale. La gamme de mesure est très large et un résultat est toujours accessible, même avec des produits noirs dont le seuil de floculation est réputé difficile à mesurer. Toutefois, ces résultats sont obtenus après modifications des paramètres opératoires de la méthode, qui deviennent alors une fonction de la nature du produit, ce qui est inacceptable quand la gamme des produits à analyser est très variable, comme dans l'industrie du pétrole.

Le document FR2655909 décrit un dispositif de détection d'un produit en suspension, en émulsion ou sous forme de microbulles dans un liquide absorbant la lumière visible. De dispositif permet de détecter le début de la précipitation des asphaltènes dans les pétroles. Il comprend un faisceau de fibres optiques dont une partie sert à l'émission et l'autre partie à la détection. Un hublot situé à proximité de l'extrémité des fibres optiques isole celles-ci du milieu à étudier. Un miroir baignant dans le milieu à mesurer permet de renvoyer le faisceau lumineux émis vers les fibres optiques servant à la détection. Ce dispositif comprend en outre des moyens de réglage de la distance séparant le hublot du miroir d'éviter la saturation ou d'obtenir le signal le plus intense possible. Ce dispositif est ainsi un dispositif à transmission indirecte (présence du miroir) et nécessite de faire varier le chemin optique pour éviter la saturation et obtenir un signal de détection le plus intense possible.

Le document US10422782B2 se rapporte à la détection de contaminants dans l'eau en faisant réagir le contaminant par réaction avec un réactif qui induit un changement de couleur et/ou une fluorescence. Le dispositif permet une mesure par absorbance et fluorescence. Le système en lui-même comprend un émetteur de lumière et un détecteur de lumière qui reçoit directement la lumière émise après qu'elle ait traversé l'échantillon. Il est prévu de moduler la source lumineuse à fréquence fixe dont le principe consiste à alimenter l'émetteur lors d'une courte période et à regarder la valeur sur le récepteur uniquement durant cette période, d'où l'utilisation d'un démodulateur configuré en "lock in amplifier". L'acquisition du signal détectée est ainsi effectuée de façon synchrone avec l'émetteur et est bloquée le reste du temps. Il s'agit ici de limiter la sensibilité au bruit et principalement à celui généré par la lumière ambiante et non de moduler l'intensité énergétique de l'émetteur comme dans la présente invention.

Le document WO2005003754A2 décrit un appareil de dosage automatique pour déterminer l'incompatibilité de produits pétroliers. Le système de détection consiste en un spectromètre de transmission de lumière par fibre optique, le liquide à mesurer passant dans une cellule optique de $100\mu m$ d'épaisseur qui n'est pas détaillée. L'appareil est équipé d'un circuit relié à plusieurs réservoirs et pompes permettant l'introduction d'un produit pétrolier, d'un solvant aliphatique, d'un solvant aromatique et d'un solvant auxiliaire dans un récipient de mélange thermostaté utilisé pour le dosage. Le temps de mesure d'un échantillon est de 1 à 2 heures.

La publication « Flocculation Onset Titration of Petroleum Asphaltènes » (Energy & Fuels, 1999, 13, pages 315-322) étudie la floculation des asphaltènes par dosage automatique utilisant notamment un spectromètre Beckmann.

Il existe aujourd'hui une norme (ASTM D7157-18 -Révision 2018) pour la détermination des valeurs S, Sa, So, qui peut être mise en œuvre au moyen d'un dispositif et d'un procédé décrits dans le document EP1751518 B1.

Le document EP1751518 B1 décrit un procédé de mesure du seuil de floculation dans lequel on introduit au moins deux sondes à émetteur et récepteur de lumière dans le milieu à mesurer, ces sondes opérant par transmission optique au niveau de zones de détection de dimensions distinctes. On détermine ensuite laquelle des deux sondes est appropriée à la mesure par détermination du seuil de transmission du milieu avant addition de solvant aliphatique. On détermine enfin à l'aide de la sonde ainsi désignée la floculation après addition de la quantité de solvant aliphatique nécessaire à la floculation. En particulier, l'une des sondes fonctionne en transmission indirecte par réflexion. Le procédé et le dispositif décrits permettent de choisir parmi plusieurs sondes introduites dans un même milieu la sonde la plus adaptée à la mesure, en particulier après ajout de solvant aliphatique. Ainsi, le dispositif peut passer d'une sonde à l'autre après ajout de solvant. Ces changements de sonde, qui correspondent à des changements du chemin optique parcouru par le faisceau lumineux entre un émetteur et son récepteur, sont simples et rapides mais peuvent provoquer des phénomènes d'oscillations du signal susceptibles d'induire des erreurs de détermination du seuil de floculation. La société ROFA® commercialise une sonde présentant un chemin optique de longueur réglable (sonde SVA-130®) qui pourrait permettre d'éviter de telles oscillations. Toutefois, la modification de la longueur du chemin optique nécessite l'intervention d'un opérateur ce qui allonge considérablement le temps de mesure.

Les méthodes aujourd'hui proposées pour la mesure du seuil de floculation des asphaltènes dans des produits hydrocarbonés, présentent donc un certain nombre d'inconvénients. Elles n'offrent pas nécessairement la simplicité, la rapidité et la précision requises des résultats, notamment pour un pilotage en continu d'une unité de traitement, par exemple de viscoréduction et/ou d'une unité de mélanges efficace. Elles ne permettent pas, non plus, l'analyse directe d'une large gamme de produits selon leur teneur en asphaltènes. Elles utilisent des techniques qui ne sont pas facilement automatisables et/ou qui ne sont pas d'une grande simplicité d'utilisation.

La présente invention vise à remédier à un ou plusieurs des inconvénients mentionnés ci-dessus.

RESUME DE L'INVENTION

L'invention a pour objet un dispositif de mesure du seuil de floculation d'un milieu colloïdal par addition de solvant aliphatique, comprenant :

- au moins une cellule de mesure fonctionnant par transmission optique directe et présentant une chambre de mesure destinée à recevoir le milieu, et, associé à chaque cellule de mesure :

    - un émetteur de lumière émettant un faisceau lumineux entrant dans la chambre de mesure suivant une direction d'émission,
    - un récepteur de lumière photoélectrique recevant directement le faisceau lumineux sortant de la chambre de mesure, le récepteur étant apte à délivrer un courant lorsqu'il reçoit un flux lumineux,
    - un système de commande comprenant :

        un système de pilotage de l'émetteur de lumière configuré pour faire varier l'intensité lumineuse du faisceau lumineux émis entre une valeur minimale et une valeur maximale,
        un système de mesure du courant délivré par le récepteur de lumière comprenant :

            un convertisseur courant-tension recevant le courant délivré par le récepteur de lumière et délivrant une tension, ce convertisseur comprenant un commutateur commandé distribuant le courant dans un circuit choisi parmi au moins deux circuits d'impédance présentant des impédances différentes,
            un amplificateur à gain variable recevant la tension délivrée par le convertisseur courant-tension et délivrant une tension,
            un convertisseur analogique-numérique recevant la tension délivrée par l'amplificateur à gain variable et délivrant un signal numérique représentatif de la quantité de courant délivrée par le récepteur de lumière,

- un système de gestion du système de commande de chaque cellule de mesure, configuré pour commander le système de pilotage de l'émetteur de lumière, le commutateur du convertisseur courant-tension et l'amplificateur à gain variable de chaque système de commande.

Cette configuration du système de commande et de gestion permet de moduler l'intensité lumineuse du faisceau lumineux émis par l'émetteur en fonction du milieu à mesurer et de sélectionner une gamme d'amplitude du signal reçu dans la zone de détection du convertisseur analogique-numérique du système de mesure. Ainsi, il est possible de moduler la gamme de mesure du signal et d'obtenir une détection, sans saturation, pour tout type d'échantillon clair ou foncé.

Le dispositif de mesure selon l'invention présente l'avantage de permettre la mesure d'échantillons très clairs à très foncés sans nécessiter une modification du chemin optique, que ce soit par déplacement de pièces mobiles de la cellule de mesure ou par l'utilisation de cellules distinctes présentant des chemins optiques différents.

En outre, la cellule de mesure fonctionne par transmission optique directe, autrement dit le faisceau lumineux émis par l'émetteur est reçu directement par le récepteur, sans dispositif optique intermédiaire de réflexion.

Notamment, avantageusement, chaque cellule de mesure peut ainsi présenter une chambre de mesure définie par des parois fixes dont deux parois opposées formant des éléments optiques aptes à être traversés par un faisceau lumineux. Dès lors, la longueur du chemin optique de la cellule de mesure est fixe, la cellule de mesure ne comprend donc pas de pièces mobiles, l'émetteur et le récepteur étant fixes. De manière générale, l'émetteur et le détecteur associés sont avantageusement situés en dehors de la chambre de mesure, chacun en regard d'un élément optique de la chambre de mesure.

Ainsi, une seule sonde est nécessaire pour mesurer des produits plus ou moins foncés qui laissent par conséquent passer plus ou moins le faisceau lumineux émis, ce qui permet d'éviter les oscillations observées avec le dispositif décrit dans le document EP1751518 B1.

L'utilisation d'un système de gestion permet une automatisation du fonctionnement du dispositif et ainsi de s'affranchir d'un opérateur.

On notera que le système de gestion peut être relié à d'autres éléments du dispositif et agencé pour les commander/contrôler, tels que des capteurs de température, un ou plusieurs organes de régulation de température du milieu, ou encore des électrovannes, voire des organes de mise en circulation de fluides, pour contrôler la distribution de fluides et éventuellement leur circulation, notamment lorsque le dispositif comprend un circuit tel que décrit plus bas.

Le système de pilotage de l'émetteur permet de faire varier l'intensité lumineuse du faisceau lumineux, autrement dit la quantité de lumière émise dans la direction d'émission. S'agissant d'un émetteur pouvant notamment émettre dans l'infra-rouge ou le proche infra-rouge, on comprendra que par « intensité lumineuse », on entend ici par abus de langage l'intensité énergétique, à savoir une grandeur radiométrique qui est la mesure de la puissance (ou flux énergétique) d'un rayonnement électromagnétique émis par une source quasi-ponctuelle, par unité d'angle solide, dans une direction donnée. Son unité dans le système international est le watt par stéradian (W sr$^{-1}$). Notamment, la variation de l'intensité lumineuse mentionnée plus haut est la variation de l'intensité énergétique (la puissance rayonnée dans le cône d'émission) obtenue en faisant varier le courant électrique traversant l'émetteur. Lorsque l'émetteur est une diode électroluminescente, l'intensité énergétique est quasiment proportionnelle au courant électrique traversant la diode.

Avantageusement, le système de pilotage peut permettre de faire varier l'intensité lumineuse par des incrémentations très faibles.

Ce système de pilotage de l'intensité lumineuse émise peut avantageusement être un système de pilotage de l'intensité du courant alimentant l'émetteur. L'émetteur est typiquement alimenté par un courant continu, dont l'intensité peut être modifiée.

On notera que le pas de variation de l'intensité du courant alimentant l'émetteur pourra être choisi par l'homme du métier en fonction de la précision de mesure souhaitée pour les produits à mesurer. Notamment, plus le pas de variation sera faible, plus la précision de mesure sera élevée, notamment sur les produits clairs. A titre d'exemple, on pourra faire varier l'intensité du courant alimentant l'émetteur dans une plage allant de quelques microampères à 100mA, pour un émetteur de type diode électroluminescente.

Ce système de pilotage peut par exemple comprendre un, ou être constitué d'un, convertisseur numérique-analogique. Le nombre de bits du convertisseur numérique-analogique pourra être choisi en fonction des variations d'intensité de courant souhaitées : plus le nombre de bits sera élevé, plus le pas de variation de l'intensité du courant sera faible. Pour une application à la mesure de produits hydrocarbonés contenant des asphaltènes, on pourra par exemple utiliser un convertisseur numérique-analogique à au moins 16 bits.

En fonction de la plage de variation d'intensités de courant de l'émetteur, le système de pilotage pourra être configuré pour faire varier l'intensité du courant par pas de l'ordre de 1 à 2μA.

L'amplificateur à gain variable peut présenter plusieurs gains de 0 à une valeur maximale G, par puissance de 2, un gain égal à 0 correspondant à l'absence de modification de l'amplitude des tensions reçue et délivrée, un gain G correspondant à une tension délivrée présentant une amplitude G fois supérieure à l'amplitude de la tension reçue. A titre d'exemple, le gain pourra varier de 0 à 128 et prendre ainsi les valeurs de 0, 2, 4, 8, 16, 32, 64 et 128.

Avantageusement, pour une réalisation simple, l'amplificateur à gain variable peut être intégré au convertisseur analogique-numérique. En particulier, le convertisseur analogique-numérique peut former un amplificateur à gain variable. Le nombre de bits du convertisseur analogique-numérique pourra être choisi en fonction de la résolution souhaitée. Un convertisseur analogique-numérique à 16 ou 24 bits peut par exemple être utilisé pour une application à la mesure de produits hydrocarbonés contenant des asphaltènes.

Avantageusement, l'impédance de chaque circuit d'impédance du convertisseur courant-tension peut être choisie de sorte que, dans une plage d'intensités de courant, la tension délivrée par l'un des circuits d'impédance présente une plage d'amplitude recoupant la plage d'amplitude de la tension délivrée par un autre circuit d'impédance. Ceci permet de couvrir l'ensemble des zones de fonctionnement.

Dans un mode de réalisation préféré, seuls deux circuits d'impédance peuvent être prévus. Ceci permet notamment de limiter les composants électroniques et les perturbations qu'ils sont susceptibles d'engendrer. On pourrait néanmoins prévoir davantage de circuits d'impédance selon les besoins.

Chaque chambre de mesure peut présenter deux éléments optiques fixes formant des parois opposées, la distance minimale séparant les deux éléments optiques dans la direction d'émission présentant une valeur comprise dans la plage de 0,4 à 1,2mm, de préférence de 0,5 à 1mm. Une telle distance est particulièrement adaptée à la mesure d'échantillons d'hydrocarbures très variés, de très clairs à très sombres, notamment des échantillons contenant des asphaltènes.

L'émetteur et le récepteur de chaque cellule de mesure peuvent respectivement présenter une ouverture de sortie du faisceau lumineux et une zone sensible. Avantageusement, ladite ouverture de sortie et ladite zone sensible peuvent chacune être positionnées à l'intérieur d'un logement étanche aux rayonnements lumineux provenant de l'extérieur de la cellule de mesure, chaque logement débouchant uniquement sur la chambre de mesure, sur des parois opposées de celle-ci, notamment sur des parois formant des éléments optiques aptes à être traversés par un faisceau lumineux. Ceci permet d'améliorer la précision de mesure, notamment pour des échantillons foncés.

Avantageusement, le dispositif de mesure peut comprendre au moins un capteur de température et au moins un organe de régulation de température reliés au système de gestion et le système de gestion peut être agencé pour réguler la température du milieu.

Avantageusement, chaque cellule de mesure peut comprendre une entrée et une sortie de fluide reliant la chambre de mesure à un circuit de fluide associé équipé d'un organe de mise en circulation du fluide. Autrement dit, chaque chambre de mesure d'une cellule de mesure fait partie d'un circuit de fluide propre du dispositif de mesure selon l'invention, qui n'est pas en communication avec d'autre(s) circuit(s) de fluide.

Un tel circuit de fluide peut être formé d'une ou plusieurs conduites raccordées les unes aux autres.

En particulier, chaque circuit de fluide peut comprendre en outre un ou plusieurs des éléments suivants :

- au moins un réservoir et au moins une conduite d'injection de liquide reliée à chaque réservoir, optionnellement connectée au circuit par une vanne, notamment une électrovanne,
- une enceinte de mélange présentant une entrée et une sortie connectées au circuit de fluide,
- au moins un organe de régulation de température.

Cet organe de régulation de température peut être choisi parmi un échangeur de chaleur, une résistance de chauffage, un dispositif à effet Peltier ou autre.

Avantageusement, le circuit de fluide peut former une boucle fermée à l'intérieur de laquelle circule le milieu.

Le dispositif de mesure selon l'invention permet de réaliser des mesures en un temps très court, rendant possible des mesures en continu.

Aussi, avantageusement, le dispositif peut comprendre des moyens d'injection de liquide en continu, et notamment à débit constant, à l'intérieur du circuit de fluide, notamment à l'intérieur de conduites du circuit de fluide. Ceci permet une injection en continu du solvant aliphatique à l'intérieur du circuit. Une telle injection en continu alors que le liquide circule à l'intérieur du circuit permet d'obtenir rapidement une homogénéisation du mélange. En raison du temps très court de mesure, on peut alors réaliser une mesure au moyen de la cellule de mesure alors que le liquide circule à l'intérieur du circuit de fluide, sans cesser d'ajouter le solvant, ce dernier étant injecté avec un débit constant faible. Cette homogénéisation sera d'autant plus rapide que le solvant sera injecté à l'intérieur de conduites du circuit. Ces moyens d'injection peuvent comprendre une conduite d'injection, une pompe et une électrovanne.

Le dispositif selon l'invention pourra présenter deux ou trois cellules de mesure identiques, chacune associée à un émetteur, un récepteur et un système de commande, chaque cellule étant reliée à son propre circuit de fluide. Les systèmes de commande des cellules de mesure pourront être commandés par un même système de gestion.

L'invention a encore pour objet un procédé de mesure du seuil de floculation d'un milieu colloïdal, notamment d'un milieu colloïdal contenant des asphaltènes, par addition de solvant aliphatique mis en œuvre par le dispositif selon l'invention, comprenant les étapes suivantes :

(i) on introduit le milieu à l'intérieur de la chambre de mesure de la cellule de mesure fonctionnant par transmission optique directe du dispositif de mesure,
(i1) optionnellement, une étape de dilution dudit milieu avec une quantité prédéterminée de solvant aliphatique préalablement à l'étape (i),
(ii) à l'aide du système de gestion du dispositif de mesure du seuil de floculation, on règle une intensité lumineuse du faisceau lumineux émis par l'émetteur, on commande le commutateur du convertisseur courant-tension pour sélectionner un circuit d'impédance et on sélectionne un gain de l'amplificateur à gain variable de manière à obtenir un signal détectable par le convertisseur analogique-numérique,
(iii) on détermine le seuil de floculation à l'aide du dispositif de mesure après addition de la quantité de solvant aliphatique nécessaire à la floculation, optionnellement on modifie le gain de l'amplificateur à gain variable du dispositif de mesure en cours d'addition, optionellement, le solvant aliphatique est ajouté en continu, et notamment à débit constant, et on procède sures à l'aide du dispositif de mesure alors que le solvant aliphatique est en cours d'addition.

Avantageusement, pendant la mesure, l'intensité lumineuse du faisceau lumineux et le circuit d'impédance ne sont pas modifiés, seul le gain pouvant être réglé. Une étape de réglage de l'intensité lumineuse émise par l'émetteur, du circuit d'impédance et du gain pourra alors être prévue avant le début de la mesure. Notamment, avant le début de la mesure, on pourra avantageusement régler le gain à une valeur maximale ou une valeur non minimale, qui pourra ainsi être réduite au fur et à mesure de la dilution de l'échantillon et de son éclaircissement.

Le dispositif de mesure du seuil de floculation selon l'invention permet de réaliser des mesures dans un temps très court, de l'ordre de quelques microsecondes, autorisant la prise de mesures alors que le solvant aliphatique est en cours d'addition. Ainsi, avantageusement, lors de l'étape (iii), le solvant aliphatique peut être ajouté en continu et on procède aux mesures à l'aide du dispositif de mesure alors que le solvant aliphatique est en cours d'addition. En particulier, le solvant aliphatique peut alors être injecté à l'intérieur d'un circuit de fluide, notamment à l'intérieur de conduites de ce dernier, le circuit de fluide étant relié à la chambre de mesure de la cellule de mesure, ce circuit de fluide étant équipé d'un organe de mise en circulation du fluide.

Avantageusement, ces mesures en continu pourront être réalisées à débit constant de solvant aliphatique.

Avantageusement, les sondes peuvent être des sondes émettant dans le domaine du NIR et on détermine l'occurrence de la floculation par détermination du pic d'absorption.

Avantageusement, le procédé peut être mis en œuvre à une température prédéterminée réglable, par exemple au moyen d'un organe de régulation de la température. Ceci peut permettre de chauffer le produit, par exemple pour faciliter sa dissolution, par exemple avant addition du solvant aliphatique, mais de réaliser la mesure à une température prédéterminée plus basse. A titre d'exemple, la mesure pourra être réalisée à une température de 15 à 60°C.

Avantageusement, le procédé peut comprendre une étape de dilution (i1) du milieu colloïdal avec une quantité prédéterminée de solvant aliphatique préalablement à l'étape (i).

Selon un mode de réalisation, le milieu colloïdal comprend des asphaltènes.

L'invention fournit encore un procédé de détermination de la stabilité d'un mélange comprenant des asphaltènes par mise en œuvre au moins deux fois du procédé de mesure du seuil de floculation d'un milieu colloïdal selon l'invention sur un milieu contenant le mélange et une quantité donnée de solvant aromatique, à des taux de dilution différents. Le procédé de mesure du seuil de floculation peut notamment être mis en œuvre au moins deux fois successivement dans une même cellule de mesure d'un dispositif de mesure ou être mis en œuvre simultanément dans deux ou plusieurs cellules de mesure identiques d'un même dispositif de mesure.

Selon un mode de réalisation, le couple solvant aromatique/solvant aliphatique (notamment paraffinique) utilisé est le couple toluène/n-heptane.

Les différents modes de réalisation précédemment décrits ainsi que ceux décrits en référence aux figures pourront être combinés.

BREVE DESCRIPTION DES FIGURES

La figure 1 est une représentation du graphe aromaticité du solvant en fonction de l'inverse de la dilution, c'est à dire la courbe de précipitation d'un produit noir qui, à un taux de dilution déterminé de ce même produit noir, associe l'aromaticité minimale du solvant nécessaire pour que le mélange ne précipite pas.
La figure 2 est une représentation schématique d'un dispositif selon un mode de réalisation de l'invention.
La figure 3 est une représentation schématique en perspective et en coupe d'une cellule de mesure selon un mode de réalisation du dispositif de l'invention.
La figure 4 est une représentation schématique en coupe d'une partie de la cellule de mesure de la figure 3.
La figure 5 est une représentation schématique d'un système de commande d'une cellule de mesure.
Les figures 6a, 6b et 6c représentent respectivement les valeurs S, Sa et So du produit noir E1 en fonction du nombre d'essais.
La figure 7 représente les valeurs S du produit noir E2 en fonction du nombre d'essais.

EXPOSE DETAILLEE DES MODES DE REALISATION DE L'INVENTION

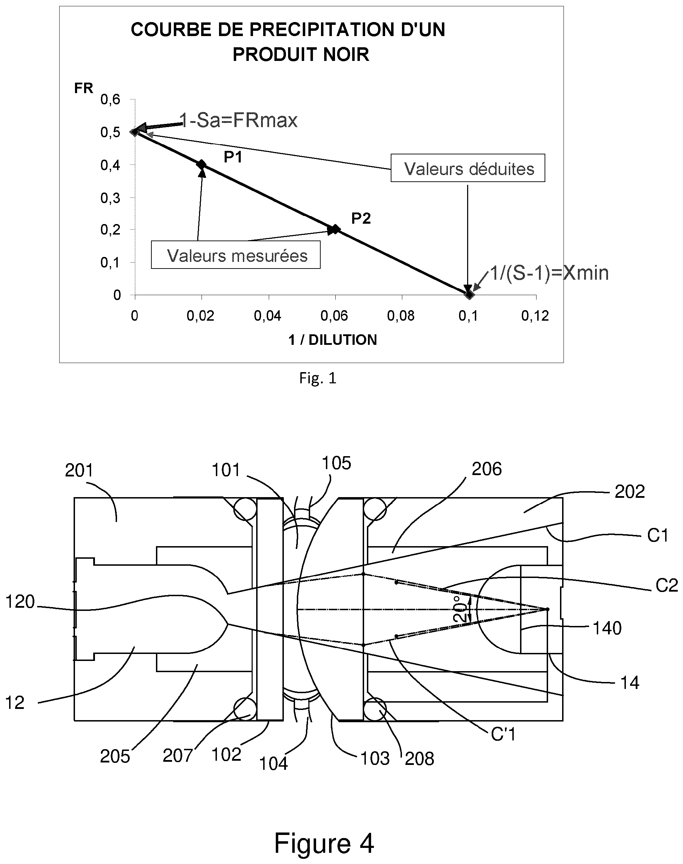

En référence à la figure 1, on décrit la méthode utilisant le dispositif décrit dans le document EP1751518 B1 pour la détermination des valeurs de S, So et Sa, pour un mélange donné de produit noir.

La stabilité intrinsèque de tout système colloïdal est quantifiée par une dilution à l'aide d'un solvant paraffinique d'un produit noir, tel qu'un fioul, un résidu de distillation atmosphérique du pétrole (ou sous vide), un pétrole brut, préalablement mélangé avec un solvant aromatique. Cette stabilité intrinsèque (S) dépend du caractère aromatique des asphaltènes (Sa) et du caractère aromatique du milieu (So), comme il a été décrit plus haut. On détermine ainsi la stabilité intrinsèque S d'un système colloïdal par mesure du seuil de floculation d'au moins 2 mélanges différents. A partir d'au moins ces 2 points, on trace une droite, dite de précipitation d'un produit noir (fig 1), qui permet d'accéder aux paramètres Sa et S, puis par calcul, à la valeur So.

Par ajout d'un solvant paraffinique dans le produit noir, le mélange devient instable à partir d'un certain taux de dilution Xmin, appelé "taux de dilution minimal".

On utilise les définitions suivantes, telles que définies dans la norme ASTM D7157-18 (Révision 2018) :

- Taux de dilution X (ml/g):
  volume de solvant total (aromatique + paraffinique) en millilitres/masse de produit noir en grammes.
- stabilité intrinsèque S du produit noir :
  S = 1 + Taux de dilution minimal. On retrouve ici la notion de S-1 comme réserve de stabilité.

Pour les mesures expérimentales, on utilise deux types de solvants, le premier est aromatique, constitué essentiellement de molécules aromatiques pour la dilution de l'échantillon (par exemple toluène, xylène, ou encore 1-méthylnaphtalène) et le second est aliphatique de type solvant paraffinique (par exemple n-heptane, cétane, ou encore iso-octane) pour provoquer la floculation des asphaltènes.

Le taux de floculation FR (« flocculation ratio ») est défini comme suit :
FR=volume de solvant aromatique/volume de solvant total.

L'aptitude des asphaltènes à être peptisés (« peptizability of an asphaltene ») est définie par : Sa=1 - FRmax, où FRmax est le taux de floculation maximal (à 1/X =0).

On appelle courbe de précipitation la fonction du taux de floculation FR en fonction du taux de dilution, soit ici :

$$1-Sa=f(1/X)=A + B/X.$$

A et B sont des constantes qui dépendent uniquement de l'échantillon et permettent d'accéder aux valeurs de S, So et Sa.

On procède comme suit. On part d'un premier mélange d'une masse donnée de produit noir dans une quantité donnée de solvant aromatique et on ajoute par incréments successifs un solvant paraffinique. On détermine le seuil de floculation (notamment par une méthode utilisant une sonde IR) et on note alors le taux de dilution et le taux de floculation FR associé au mélange analysé. On obtient un premier point, identifié par le point P1 sur le graphe (fig 1). On recommence l'opération, avec un produit de départ qui est initialement moins fortement dilué dans le solvant aromatique. On obtient alors une autre mesure matérialisée par le point P2. Avec les deux points P1 et P2 il est alors possible de tracer la droite passant par ces points et d'obtenir des valeurs limites (1-Sa) sur l'axe des ordonnées (FRmax ou taux de dilution infini) et 1/(S-1) sur l'axe des abscisses (FR nul). Il devient ensuite possible d'accéder aux valeurs de S, Sa puis So par le calcul.

Cette technique, qui fait référence à la méthode normalisée ASTM D7157-18 (Révision 2018), et qui consiste en la construction d'une courbe de précipitation, à partir d'au moins deux résultats de mesures (trois dans la norme), pour ensuite déterminer les valeurs des aromaticités limites et nulles, est celle généralement suivie dans l'invention. Les masses, les volumes et les produits utilisés sont tout à fait classiques dans l'art de ce type d'analyse.

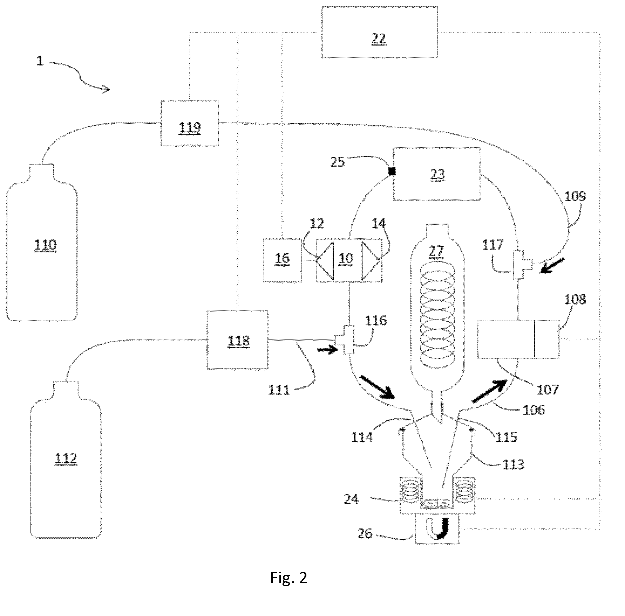

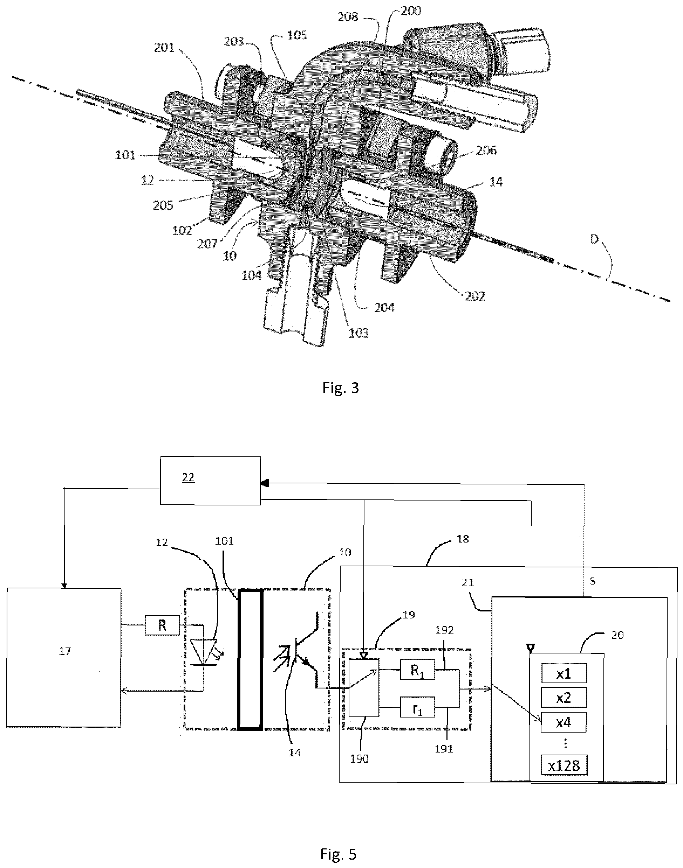

En référence à la figure 2 et aux figures 3 à 5, le dispositif (1) selon l'invention comprend une cellule de mesure (10) fonctionnant par transmission optique directe et présentant une chambre de mesure (101). Cette chambre de mesure est de dimensions fixes, définie par des parois fixes.

Le dispositif (1) comprend également, associés à la cellule de mesure (10), un émetteur de lumière (12) émettant (configuré pour émettre) un faisceau lumineux entrant dans la chambre de mesure (101) suivant une direction d'émission (D) et un récepteur de lumière (14) recevant directement le faisceau lumineux sortant de la chambre de mesure (101). Autrement dit, le récepteur de lumière (14) est positionné de manière à recevoir directement le faisceau lumineux sortant. Il peut notamment être positionné dans la direction d'émission (D), d'un côté de la chambre de mesure (101) opposé au côté où est situé l'émetteur de lumière (12), tel que représenté sur les figures. L'émetteur est par exemple un émetteur IR classique, par exemple une diode électroluminescente. L'émetteur (12) pourra de préférence être choisi de sorte que son spectre d'émission soit constant quelle que soit l'intensité lumineuse qu'il émet, autrement dit quelque soit l'intensité du courant électrique qui l'alimente. A titre d'exemple, on pourra utiliser une diode électroluminescente à base d'arséniure de gallium-aluminium.

Le récepteur (14) est un récepteur photoélectrique apte à délivrer un courant lorsqu'il reçoit un flux lumineux.

Dans l'exemple représenté figure 3, la cellule de mesure (10) comprend une entrée (104) et une sortie (105) en communication de fluide avec la chambre de mesure (101).

Dans le mode de réalisation représenté, contrairement aux sondes usuelles existantes, la chambre de mesure (101) fait partie de la cellule de mesure (10) mais n'est pas définie par l'émetteur (12) et le récepteur (14) bien qu'elle soit située entre ces derniers permettant au faisceau lumineux de traverser la chambre de mesure. La chambre de mesure (101) est ici définie par des parois fixes dont deux parois opposées formant des éléments optiques (102, 103) aptes à être traversés par un faisceau lumineux.

Un premier élément optique (102) situé du côté de l'émetteur (12), ici une lame à faces parallèles, permet la transmission du faisceau lumineux provenant de l'émetteur (12) à l'échantillon situé à l'intérieur de la chambre de mesure (101). Un deuxième élément optique (103) situé du côté du détecteur (14), ici une lentille sphérique plan-convexe, permet de focaliser le faisceau lumineux transmis par l'échantillon sur le détecteur (14).

D'autres couples d'éléments optiques que ceux précédemment listés peuvent être envisagés, toutefois, la configuration de l'exemple présente l'avantage d'être particulièrement performante.

En particulier, les deux éléments optiques peuvent être choisis parmi une lame à faces parallèles et une lentille asphérique, deux lames à faces parallèles, une lame à faces parallèles et une lentille sphérique, de préférence une lame à faces parallèles et une lentille sphérique.

On notera que chacun des éléments optiques (102, 103) peut être une lentille sphérique, une lame à faces parallèles ou une lentille asphérique. Ces différents éléments optiques peuvent être réalisés en verre, polymère, métalloïde, mais également en matière hybride (verre/polymère).

La cellule de mesure (10) peut être réalisée en matériau polymère et formée par exemple d'un corps (200) définissant l'entrée (104), la sortie (105) et les parois de la chambre de mesure (101) qui ne sont pas formées par les éléments optiques (102), (103). L'émetteur (12) et le récepteur (14) sont ici disposés chacun à l'intérieur de supports (201), (202), ici de forme cylindrique avec des collerettes de fixation, insérés dans des orifices correspondants (203), (204) du corps. Ces supports (201), (202) sont situés de part et d'autre de la chambre de mesure (101) suivant la direction D. Ces supports (201), (202), lorsqu'ils sont solidarisés au corps (200), maintiennent les éléments optiques (102) et (103), insérés au fond des orifices (203), (204) en position de vis-à-vis à l'intérieur du corps. Des joints toriques (207, 208) disposés entre chaque élément optique (102, 103) et le support (201, 202) qui le maintient en position permettent d'étanchéifier la chambre de mesure (101) et d'assurer un bon maintien des éléments optiques.

La chambre de mesure (101) peut se présenter sous la forme d'une conduite ouverte à ses deux extrémités, à section transversale fermée.

L'invention n'est bien entendu pas limitée par une forme particulière de la cellule de mesure, pourvu que les parois de la chambre de mesure soient fixes et que l'émetteur et le récepteur soient situés en dehors de la chambre de mesure. De manière générale, la cellule de mesure utilisée dans la présente invention ne contient aucun élément mobile ou déplaçable, y compris l'émetteur et le récepteur.

On choisira de préférence une distance séparant les éléments optiques (102), (103) suffisamment faible pour permettre la détection d'échantillons très foncés. Avantageusement, pour des hydrocarbures susceptibles de contenir des asphaltènes, on pourra fixer la distance minimale séparant les deux éléments optiques dans la direction d'émission D à une valeur comprise dans la plage de 0,4 à 1,2mm, de préférence de 0,5 à 1mm.

La figure 4 représente schématiquement le chemin parcouru par le faisceau lumineux selon un mode de réalisation. Sur cette figure, le récepteur (14), et plus précisément sa zone sensible, est positionné au point focal théorique du système optique, les éléments optiques (102), (103) de la chambre de mesure (101) étant séparés d'une distance choisie dans la plage de valeur de 0,4 à 1,2mm.

Dans cet exemple, le cône d'émission C1' de l'émetteur (12), correspondant au cône C1 de faisceau lumineux émis par l'émetteur après qu'il ait traversé les deux éléments optiques (102, 103), présente ici un demi-angle au sommet de 10 à 15°, le demi-angle au sommet du côté de réception C2 du récepteur (14) étant de 10°. On notera ici que le cône de réception est entièrement contenu à l'intérieur du cône d'émission lumineuse de l'émetteur, et n'en sort pas.

L'homme du métier pourra avantageusement configurer la cellule de mesure afin que le cône d'émission C1 de l'émetteur éclaire un volume suffisant de la chambre de mesure (101) pour que la quantité de produit éclairée par ce cône d'émission C1 soit homogène et représentative du produit à mesurer.

Dans l'exemple représenté, l'émetteur (12) et le récepteur (14) de chaque cellule de mesure présentent respectivement une ouverture de sortie (120) du faisceau lumineux et une zone sensible (140), lesquelles sont chacune respectivement positionnées à l'intérieur d'un logement (205, 206) étanche aux rayonnements lumineux provenant de l'extérieur de la cellule de mesure. Chaque logement (205, 206) débouche uniquement sur la chambre de mesure (101), sur les parois opposées de celle-ci formées par les éléments optiques (102, 103). Dit autrement, chaque logement (205, 206) est fermé par un élément optique (102, 103) de la chambre de mesure (101). Dans l'exemple, ces logements (205, 206) font partie des supports (201, 202) décrits précédemment.

Le dispositif (1) comprend également un système de commande (16) et un système de gestion (22) du système de commande.

Le système de commande (16) comprend un système de pilotage (17) de l'émetteur de lumière et un système de mesure (18) du courant délivré par le récepteur de lumière.

Le système de pilotage (17) de l'émetteur de lumière est configuré pour faire varier l'intensité lumineuse du faisceau lumineux émis par l'émetteur (12) entre une valeur minimale et une valeur maximale. Dans l'exemple représenté, il s'agit d'un système de pilotage de l'intensité du courant alimentant l'émetteur. On pourra avantageusement utiliser un convertisseur numérique-analogique à 16 bits. Un tel convertisseur permet de moduler finement la variation d'intensité du courant électrique qui alimente l'émetteur (12). Cette modulation est fonction du nombre de points maximum du convertisseur (ici $2^{16}=65536$ points au maximum). Une telle plage dynamique permet d'alimenter l'émetteur avec un courant très faible (par exemple de l'ordre de quelques micro-ampères pour un nombre de points inférieur à 200), correspondant à une faible intensité lumineuse émise, jusqu'à un courant fort (près de 92mA pour le nombre maximal de points), et donc une intensité lumineuse maximale. L'invention n'est bien entendu pas limitée à ce mode de réalisation et l'on pourrait utiliser un logiciel pour moduler l'intensité du courant sur une large plage avec une précision élevée ou tout autre dispositif adapté. L'utilisation d'un convertisseur numérique-analogique à 16 bits présente néanmoins l'avantage d'être simple et robuste. Bien entendu, un convertisseur présentant davantage de bits pourrait être utilisé. On notera que le système de pilotage fournit un courant continu à l'émetteur et non un courant pulsé.

Tel que représenté figure 5, le système de mesure (18) comprend un convertisseur courant-tension (19), un amplificateur à gain variable (20) et un convertisseur analogique-numérique (21).

Le convertisseur courant-tension (19) reçoit le courant délivré par le récepteur (14) et délivre une tension. Ce convertisseur (19) comprend un commutateur commandé (190) distribuant le courant dans un circuit choisi parmi au moins deux circuits d'impédance présentant des impédances différentes. Sur la figure, le convertisseur courant-tension (19) présente un premier circuit d'impédance (191) présentant une impédance r1 et un deuxième circuit d'impédance (192) présentant une impédance R1, supérieure à l'impédance r1. Le convertisseur courant-tension (19) permet ainsi d'obtenir deux gammes de mesure. Lorsque l'échantillon à mesurer est clair, autrement dit absorbant peu la lumière, le système de gestion (22) peut être configuré pour commander le commutateur (190) et distribuer le courant au premier circuit (191) de faible impédance afin d'éviter de saturer le convertisseur analogique-numérique (21) en aval. A l'inverse, lorsque l'échantillon à mesurer est foncé, autrement dit absorbant fortement la lumière, le système de gestion (22) peut être configuré pour commander le commutateur (190) et distribuer le courant au deuxième circuit (192) de plus forte impédance afin de générer une tension détectable par le convertisseur analogique-numérique (21) en aval.

La tension délivrée par le convertisseur courant-tension (19) entre ensuite dans le convertisseur analogique-numérique (21) via l'amplificateur à gain variable (20). Ce dernier reçoit ainsi la tension délivrée par le convertisseur courant-tension (19) et délivre à son tour une tension égale ou proportionnelle à la tension entrante. L'amplificateur à gain variable (20) présente plusieurs gains (ici de 0 à 128 par puissance de 2) qui agissent comme autant de gammes de mesure supplémentaires. En début de mesure, le système de gestion (22) peut être configuré pour sélectionner un gain suffisamment élevé, et de préférence non minimal, et pour, en cours de mesure, réduire ce gain pour éviter une saturation du convertisseur analogique-numérique (21) au fur et à mesure de la dilution du produit testé. L'homme du métier pourra ainsi déterminer le nombre de gains nécessaires en fonction des produits à tester, afin que la tension délivrée par l'amplificateur à gain variable (20) soit toujours dans la plage d'utilisation du convertisseur analogique-numérique (21) utilisé.

Enfin, le convertisseur analogique-numérique (21) reçoit la tension délivrée par l'amplificateur à gain variable (20) et délivre un signal numérique S représentatif de la quantité de courant délivrée par le récepteur (14). On choisira de préférence un convertisseur analogique-numérique (21) à haute résolution, par exemple à 24 bits. Bien entendu, un convertisseur analogique-numérique présentant un nombre de bits différent peut être envisagé.

Le système de gestion (22) du système de commande (16) est configuré pour commander le système de pilotage de l'émetteur de lumière, le commutateur du convertisseur courant-tension et l'amplificateur à gain variable. Il permet ainsi une automatisation de la mesure.

Ce système de gestion (22) peut comprendre un ou plusieurs processeurs de type microprocesseur, microcontrôleur ou autre, par exemple faisant partie d'un ordinateur. Le ou les processeurs comportent notamment des moyens d'exécution de programme d'ordinateur adaptés pour mettre en œuvre le procédé décrit dans la présente invention.

Dans un mode de réalisation, le système de gestion peut être agencé pour recevoir des données. Le système de gestion peut également être agencé pour transmettre des données, notamment vers un dispositif d'affichage tel qu'un écran. Le système de gestion peut ainsi comprendre une ou plusieurs interfaces d'entrée, de sortie, ou d'entrée/sortie. Il peut s'agir d'interfaces de communication sans fil (Bluetooth, WIFI ou autre) ou de connecteurs (port réseau, port USB, port série, port Firewire®, port SCSI ou autre).

Dans un mode de réalisation, le système de gestion peut comporter des moyens de mémorisation qui peuvent être une mémoire vive ou une mémoire RAM (de l'anglais « Random Access Memory »), une EEPROM (de l'anglais « (Electrically-Erasable Programmable Read-Only Memory »), une mémoire flash, une mémoire externe, ou autre. Ces moyens de mémorisation peuvent notamment mémoriser les données reçues, et éventuellement de(s) programme(s) d'ordinateur.

Le système de gestion (22) est par exemple configuré pour asservir les paramètres du système de commande en fonction de l'opacité du produit testé. Cet asservissement va donc dépendre :

- de l'intensité lumineuse émise par l'émetteur,
- du circuit d'impédance sélectionné,
- du gain de l'amplificateur à gain variable,
- de la valeur initiale du signal numérique généré par le convertisseur analogique-numérique.

Cet asservissement pourra être configuré de manière à moduler l'amplitude de la tension entrant dans le convertisseur analogique-numérique afin d'atteindre une valeur de consigne correspondant à une valeur minimale mesurable par le convertisseur.

De manière connue, un convertisseur analogique-numérique peut détecter une tension dans une plage de détection déterminée : en dessous de la valeur minimale de cette plage, aucun signal n'est généré, au dessus de la valeur maximale de la plage, la saturation du convertisseur entraîne une perte de sensibilité. La valeur de consigne est généralement choisie dans une partie de la plage de détection proche de la valeur minimale.

Le système de gestion peut par exemple être configuré pour réaliser un réglage du dispositif de mesure lors d'une étape de réglage.

A titre d'exemple, cette étape de réglage peut être réalisée selon les étapes de programme décrites ci-après.

STEP 0 (étape initiale) : on sélectionne le circuit d'impédance présentant l'impédance la plus basse, on sélectionne le gain le plus élevé, on règle l'intensité lumineuse émise par l'émetteur à une valeur proche de sa valeur minimale et on enregistre la valeur du signal S délivré par le convertisseur analogique-numérique.

STEP 1 : on compare le signal S enregistré à une valeur de consigne.

Si la valeur du signal S est inférieure à la valeur de consigne, on va à l'étape STEP 2.

Si la valeur du signal S est supérieure à une valeur de consigne, on va à l'étape STEP 3.

Si la valeur du signal S est égale à la valeur de consigne, on va à l'étape STEP 4.

STEP 2 : on augmente l'intensité lumineuse émise par l'émetteur jusqu'à atteindre la valeur de consigne du signal S généré par le convertisseur ou jusqu'à atteindre l'intensité lumineuse maximale de l'émetteur.

Si la valeur de consigne du signal S est atteinte, on va à l'étape STEP 4.

Sinon, on va à l'étape STEP 5.

STEP 3 : on réduit l'intensité lumineuse émise par l'émetteur jusqu'à atteindre la valeur de consigne du signal S généré par le convertisseur ou jusqu'à atteindre l'intensité lumineuse minimale de l'émetteur.

Si la valeur de consigne du signal S est atteinte, on va à l'étape STEP 4.

Sinon, on va à l'étape STEP 9.

STEP 4 : on enregistre la valeur d'intensité lumineuse, le circuit d'impédance choisi et le gain et on va à l'étape STEP 10.

STEP 5 : on change le circuit d'impédance et on sélectionne le circuit d'impédance plus forte, on enregistre la valeur du signal et on va à l'étape STEP 6.

STEP 6 : on compare le signal S enregistré à une valeur de consigne.

Si la valeur du signal S est inférieure à la valeur de consigne, on va à l'étape STEP 7.

Si la valeur du signal S est supérieure à une valeur de consigne, on va à l'étape STEP 8.

STEP 7 : on augmente l'intensité lumineuse émise par l'émetteur jusqu'à atteindre la valeur de consigne du signal S généré par le convertisseur.

Quand la valeur de consigne du signal S est atteinte, on va à l'étape STEP 4.

STEP 8 : On réduit l'intensité lumineuse émise par l'émetteur jusqu'à atteindre la valeur de consigne du signal S généré par le convertisseur ou jusqu'à atteindre l'intensité lumineuse minimale de l'émetteur.

Si la valeur de consigne du signal S est atteinte, on va à l'étape STEP 4.

Sinon, on va à l'étape STEP 9.

STEP 9 : On réduit la valeur du gain jusqu'à atteindre la valeur de consigne du signal S généré par le convertisseur.

Quand la valeur de consigne du signal S est atteinte, on va à l'étape STEP 4.

STEP 10 : fin du programme.

La valeur de consigne correspond par exemple à une valeur minimale mesurable par le convertisseur (21).

Dans le mode de réalisation représenté, tel que visible figure 2, le dispositif (1) comprend en outre un organe de régulation de température (23) situé en amont de la cellule de mesure (10) par rapport au sens de circulation du fluide, ici un dispositif à effet Peltier pouvant être organe de refroidissement ou de chauffage selon le sens du courant électrique qui le traverse. Il comprend également un ou plusieurs capteurs de température (25), par exemple un capteur de température situé en amont de la cellule de mesure, ici en sortie de l'organe de régulation de température. On pourra également prévoir un capteur de température au niveau d'une enceinte de mélange (113), à l'entrée ou à la sortie de celle-ci, ou encore intégré à un bloc thermostaté (24) entourant cette enceinte de mélange, tel que représenté Ce bloc thermostaté (24) forme un autre organe de régulation de température ici dédié au chauffage. Ces éléments peuvent être contrôlés par le système de gestion (22) lequel peut alors être agencé pour une gestion automatique de la température du milieu. On notera que l'on pourra utiliser tout autre dispositif approprié à la régulation de température.

La cellule de mesure (10) pourrait être plongée dans le milieu de manière à ce que ce dernier remplisse entièrement la chambre de mesure. Toutefois, de préférence, tel que représenté sur les figures 2 à 4 et déjà décrit, la cellule de mesure (10) comprend une entrée

(104) de fluide et une sortie (105) de fluide reliant la chambre de mesure (101) à un circuit de fluide (106), lequel est équipé d'un organe de mise en circulation du fluide (107), ici une pompe péristaltique (107) commandée par un moteur pas à pas (108).

Plus particulièrement, dans l'exemple, le circuit de fluide (106) comprend :

- une première conduite d'injection (109) de liquide reliée à un réservoir (110) pour l'injection d'un premier solvant, par exemple le solvant aromatique,
- une deuxième conduite d'injection (111) de liquide reliée à un deuxième réservoir (112) pour l'injection d'un deuxième solvant, par exemple le solvant paraffinique,
- une enceinte de mélange (113) présentant une entrée (114) et une sortie (115) connectées au circuit de fluide (106), pour recevoir le milieu,

- l'organe de régulation de température (23) et l'organe de chauffage (24) précédemment mentionnés.

Les conduites d'injection (109) et (111) peuvent être équipées d'électrovannes (116), (117), et de pompes (118), (119) lesquelles sont de préférence commandées par le système de gestion (22) pour une automatisation du dispositif.

Le circuit de fluide (106) forme ici une boucle qui peut donc être fermée pour la circulation du milieu à l'intérieur de la boucle par exemple dans le sens de circulation symbolisé par les flèches sur la figure 2.

On pourra éventuellement prévoir une colonne de reflux (27) pour permettre de chauffer à reflux le produit contenu dans l'enceinte afin de faciliter la dissolution de l'échantillon.

Le fonctionnement du dispositif selon l'invention est décrit ci-après.

On introduit l'échantillon à analyser à l'intérieur de la chambre de mesure de la cellule de mesure du dispositif selon l'invention. Dans le dispositif représenté, on introduit l'échantillon dans l'enceinte de mélange avant de le faire circuler dans le circuit et à l'intérieur de la chambre de mesure. Notamment, le volume de produit est suffisant pour remplir entièrement au moins la chambre de mesure. A titre d'exemple, le volume de la chambre de mesure peut représenter 1/10ème du volume total du circuit.

Dans l'exemple, cette étape d'introduction est suivie d'une étape d'ajout du solvant aromatique au produit pour former le milieu à analyser. L'échantillon est alors dilué par le solvant aromatique avant de circuler à l'intérieur de la chambre de mesure de la cellule de mesure.

On procède ensuite à une étape de réglage au cours de laquelle on règle l'intensité lumineuse émise par l'émetteur, on choisit le circuit d'impédance et le gain, tel que précédemment décrit. Cette étape de réglage, effectuée avant l'ajout de solvant paraffinique, c'est-à-dire avant la floculation, permet d'obtenir un signal détectable par le récepteur. On pourra par exemple mettre en œuvre l'étape de réglage précédemment décrite. Par signal détectable, on entend un signal pouvant être distingué d'un bruit de fond et qui n'est pas saturé.

On détermine enfin à l'aide du dispositif de mesure la floculation après addition de la quantité de solvant paraffinique nécessaire à la floculation. A cet effet, on ajoute progressivement le solvant paraffinique et on note la chute de transmission correspondant à la floculation des asphaltènes. Cette détermination se fait par des techniques classiques, par exemple, par mesure du pic d'absorption.

En particulier, l'intensité lumineuse émise par l'émetteur et le circuit d'impédance restent fixes au fur et à mesure de la dilution par le solvant paraffinique. Si nécessaire, on pourra diminuer le gain en cours de dilution afin de ne pas saturer le convertisseur (21). Lors de la diminution du gain à la valeur inférieure de gain, le système de gestion pourra multiplier par 2 la valeur du signal S en sortie du convertisseur (21) ce qui permettra d'éviter une variation de l'amplitude du signal due au changement de gain.

De cette façon, le signal peut être mesuré avec une bonne précision avec une seule et unique cellule de mesure réglée de manière appropriée, ce qui offre un gain de temps appréciable pour l'opérateur.

On notera que lors de la mesure, l'intensité lumineuse émise par l'émetteur reste avantageusement fixe, ce qui est obtenu par la fourniture d'un courant continu constant à l'émetteur.

La valeur minimale de la l'intensité lumineuse émise par l'émetteur correspond par exemple à une valeur en dessous de laquelle la précision de la mesure est trop faible pour permettre de distinguer un signal du bruit de fond.

Selon un mode de réalisation avantageux, mettant notamment en œuvre le dispositif décrit en référence aux figures, l'étape d'introduction comprend une phase de dissolution, au cours de laquelle on introduit le milieu à l'intérieur de l'enceinte de mélange (113), en une quantité suffisante pour remplir entièrement le circuit (106), puis on régule la température du milieu à une température de dissolution au moyen de l'organe de chauffage (24). Cette phase de dissolution est de préférence réalisée sous agitation, ici au moyen d'un agitateur magnétique (26) disposé sous l'enceinte de mélange (113). On injecte ensuite le solvant aromatique à l'intérieur de l'enceinte de mélange (113) maintenue sous agitation.

Cette phase de dissolution peut éventuellement être suivie d'une phase de pré-dilution avec le solvant paraffinique, au cours de laquelle une quantité prédéterminée de ce solvant peut être injectée dans le circuit. On opère de cette manière dans le cas de produit très aromatique et stable ou lorsque le produit est trop sombre et la puissance du détecteur atteint son maximum sans avoir détecté le volume de floculation.

On réalise ensuite une phase de refroidissement au cours de laquelle on régule la température à une température d'essai prédéterminée au moyen de l'organe de de régulation de température (23).

On procède ensuite à une phase de dosage au cours de laquelle on ajoute progressivement le solvant paraffinique. Cette addition de solvant peut être réalisée par des additions incrémentales ou par une addition en continue. On procède alors à l'acquisition et à l'enregistrement du signal du convertisseur (21) soit après chaque ajout de solvant, soit en cours d'addition du solvant. Dans ce dernier cas, le débit d'introduction du solvant à l'intérieur du circuit pourra être constant, par exemple de l'ordre de 1mL/minute. On notera que, dans tous les cas, le produit à analyser circule dans le circuit en cours d'addition du solvant et d'acquisition du signal. Cette phase de dosage peut être arrêtée par un opérateur, lorsque le volume maximal de la cellule de mélange est atteint ou lorsqu'un nombre d'additions incrémentales prédéterminé a été effectué ou lorsqu'un volume de solvant prédéterminé a été ajouté.

On peut ensuite procéder à une phase de nettoyage en faisant par exemple circuler le solvant aromatique dans le circuit.

L'invention est décrite en référence à un dispositif comprenant une unique cellule de mesure. On notera toutefois que le dispositif de l'invention peut comprendre plusieurs cellules de mesure indépendantes identiques, par exemple trois, afin de réaliser simultanément en parallèle trois essais sur un produit.

En outre, le dispositif selon l'invention permet d'obtenir un domaine d'application spectral possible pour les mesures qui est très large. Le dispositif selon l'invention est approprié pour la détermination des valeurs de S, Sa et So pour tous types de résidus et fuels et n'est pratiquement pas limité quant à la nature du milieu à tester. Comme le dispositif comprend un seul type de cellule de mesure, il est possible de procéder à plusieurs mesures en un temps moindre par rapport aux dispositifs utilisant plusieurs cellules pour mesurer un même produit. On peut ainsi effectuer 3 mesures avec une même cellule et donc obtenir 3 points de la courbe et par-là une bonne répétabilité des mesures pour S, Sa et So. Enfin, la méthode de détermination selon l'invention peut être mise en œuvre à température ambiante ou à une température prédéterminée, ce qui permet de mesurer les paramètres S, Sa et So à une température donnée et de vérifier leur évolution en fonction de la température, puisque la stabilité des asphaltènes dépend de la température.

En général, le couple solvant aromatique/solvant paraffinique utilisé dans l'invention est le couple toluène/n-heptane.

EXEMPLES

Les exemples suivants illustrent l'invention sans la limiter.

Exemple 1

Des mesures ont été effectuées sur des échantillons de produits noirs différents pour lesquels les valeurs S, Sa ont été mesurées et So calculées, d'une part avec une méthode utilisant les sondes SVA-130® proposées par la société ROFA mettant en œuvre la méthode décrite dans la norme ASTM D7157-18 (Révision 2018) ("Méthode de mesure A") et d'autre part avec le dispositif et le procédé conformes à la présente invention ("Méthode de mesure B").

Le dispositif conforme à la présente invention est du type décrit en références aux figures 2 à 5. La cellule de mesure comprend notamment une lame à faces parallèles et une lentille sphérique, la distance entre la fenêtre et la lentille étant de 0.7 mm au centre et de 2 mm sur les bords.

Le volume de la boucle de circuit est ici de 4ml. Les mesures sont réalisées alors que le fluide circule à une vitesse de 10mL/min environ. La température d'essai est ici la température ambiante, à savoir 21°C. Il est possible de chauffer le mélange solvant aromatique/produit pour accélérer la dissolution de ce dernier, notamment dans les cas des résidus sous vide. Un chauffage de 60°C à 100°C suffit à dissoudre le produit dans ce cas en quelques minutes. Dans certains cas (produits très stables), une pré-dilution au n-heptane a été réalisée avant le début des mesures afin de limiter le volume de solvant paraffinique (n-heptane) à ajouter pour obtenir la floculation.

Dans cet exemple, les produits noirs testés correspondent à :

E1 : résidu atmosphérique viscoréduit, non fluxé
E2 : fioul lourd

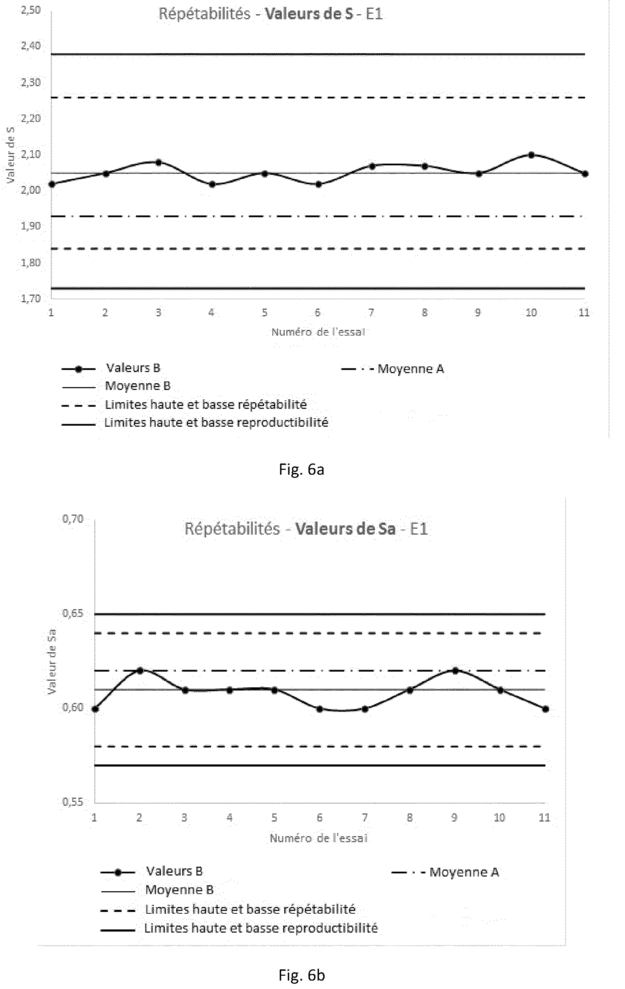

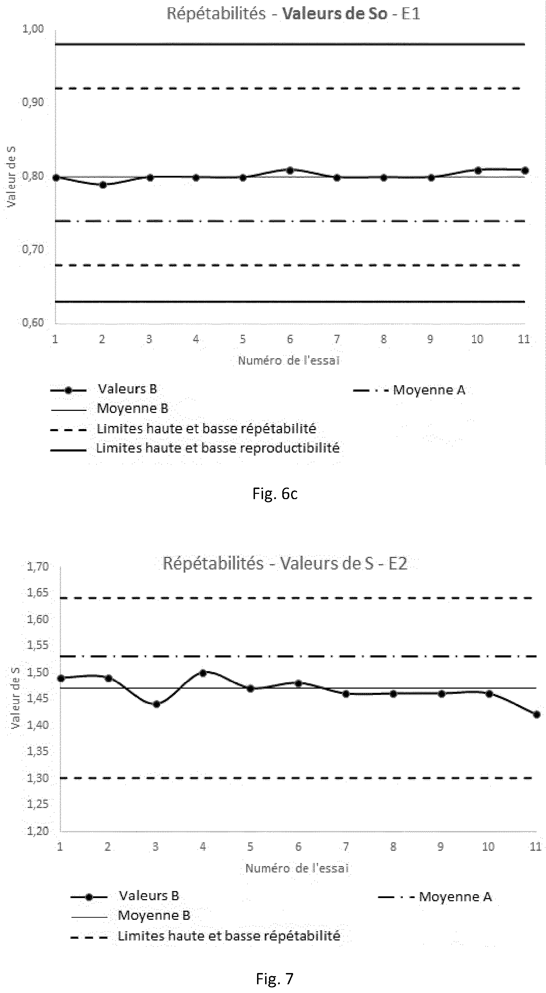

Les figures 6a, 6b et 6c représentent respectivement les valeurs S, Sa et So du produit noir E1, la figure 7 représente la valeur S du produit noir E2. Les valeurs de S, Sa et So du produit noir E2 mesurées avec la méthode selon l'invention sont rassemblées dans le tableau 1.

Tableau 1

| Essai | S | Sa | So |
|-------|------|------|------|
| 1 | 1,49 | 0,43 | 0,85 |
| 2 | 1,49 | 0,43 | 0,85 |
| 3 | 1,44 | 0,41 | 0,85 |
| 4 | 1,50 | 0,43 | 0,86 |
| 5 | 1,47 | 0,43 | 0,84 |
| 6 | 1,48 | 0,43 | 0,85 |
| 7 | 1,46 | 0,40 | 0,87 |
| 8 | 1,46 | 0,41 | 0,86 |
| 9 | 1,46 | 0,41 | 0,87 |
| 10 | 1,46 | 0,41 | 0,86 |
| 11 | 1,42 | 0,40 | 0,86 |

Chaque figure rassemble les valeurs moyennes calculées pour 11 mesures distinctes pour la méthode de mesure B selon l'invention et pour la méthode de mesure A. Les valeurs de répétabilité et de reproductibilité sont calculées en utilisant les formules figurant dans la norme ASTM D7157-18 (Révision 2018) à partir de la moyenne calculée pour les mesures de chacune des méthodes de mesure A et B.

Sur chacune de ces figures, sont représentées :

- des limites haute et basse de S, Sa et So tenant compte de la répétabilité (limites hautes et basses de répétabilité), calculées respectivement en ajoutant et en retranchant la valeur de répétabilité calculée pour la méthode de mesure B à la moyenne des mesures calculée pour la méthode de mesure B,

- des limites haute et basse S, Sa et So tenant compte de la reproductibilité (limites hautes et basses de reproductibilité), calculées respectivement en ajoutant et en retranchant la valeur de reproductibilité calculée pour la méthode de mesure B à la moyenne des mesures calculée pour la méthode de mesure B,
- la moyenne des valeurs obtenues avec la méthode de mesure B selon l'invention (Moyenne B),
- les valeurs obtenues avec la méthode de mesure B selon l'invention (valeurs B),
- la moyenne des valeurs obtenues avec la méthode de mesure A (Moyenne A).

Les courbes 6a, 6b, 6c relatives à l'échantillon E1 montrent que les valeurs S, Sa et So obtenues avec la méthode de mesure B selon l'invention sont proches des valeurs obtenues avec la méthode de mesure A, les sondes SVA-130® permettant une mise en œuvre de la norme ASTM D7157-18 (Révision 2018) dans le respect des conditions de répétabilité et de reproductibilité définies dans cette norme. De même, la courbe 7 relative à l'échantillon E2 montre que les valeurs de S obtenues avec la méthode de mesure B selon l'invention sont proches des valeurs obtenues avec la méthode de mesure A, ce qui a fortiori est également le cas des valeurs Sa et So.

Sur chaque figure 6a, 6b, 6c, 7 on constate que les minimas et les maximas des courbes des essais effectués selon la méthode de mesure B sont entre les limites basse et haute de répétabilité et de reproductibilité. Autrement dit, les écarts de valeurs entre plusieurs mesures obtenues avec la méthode de mesure B selon l'invention sont faibles pour les deux types de produits.

De plus, l'automatisation de l'analyse permet de réaliser l'analyse complète en moins d'une heure avec la méthode de mesure B selon l'invention alors qu'il faut plus de deux heures pour la méthode A en particulier en raison du temps opérateur nécessaire pour modifier le trajet optique des sondes SVA-130®. En outre, la méthode de mesure B selon l'invention est également plus rapide qu'en utilisant un dispositif et une méthode conformes au document EP1751518 B1 du fait notamment de l'automatisation de la dilution.

Pour chacune des 11 mesures réalisées avec la méthode B, le coefficient de corrélation $R^2$ de la courbe de précipitation (taux de floculation FR en fonction de l'inverse de la dilution) construite avec 3 points (P1, P2 et P3) varie:

- de 0,9952 à 0,9999 pour le produit E1,
- de 0,9936 à 0,9991 pour le produit E2.

Le coefficient $R^2$ de la courbe de précipitation est ainsi supérieur à la valeur de $R^2$ minimale (0,98) exigée par la norme.

Par ailleurs, la notion d'efficacité exprimée comme étant le rapport de la répétabilité ASTM (selon ASTM D7157-18 -Révision 2018) sur la répétabilité calculée avec la formule générale (2 x racine carrée de 2 x écart type, soit 2,83 x écart type) a été utilisée pour comparer si la répétabilité de la norme ASTM D7157-18 (Révision 2018) est plus petite ou plus grande que la répétabilité propre au dispositif selon l'invention. Notamment, plus la répétabilité est petite, plus les valeurs sont répétables et donc moins variables.

Pour le produit noir E1, cette efficacité est de 8,45, pour le produit noir E2, elle est de 7,65.

On notera ainsi que la valeur de l'efficacité de la méthode de mesure B est toujours supérieure à 1, ce qui signifie que la répétabilité du dispositif selon l'invention est plus petite que la répétabilité de la norme ASTM D7157-18 (Révision 2018).

## Revendications

1. Dispositif de mesure (1) du seuil de floculation d'un milieu colloïdal par addition de solvant aliphatique, comprenant :

   - au moins une cellule de mesure (10) fonctionnant par transmission optique directe et présentant une chambre de mesure (101) destinée à recevoir le milieu, et, associé à chaque cellule de mesure :

     - un émetteur (12) de lumière configurée pour émettre un faisceau lumineux entrant dans la chambre de mesure suivant une direction d'émission,
     - un récepteur (14) de lumière photoélectrique recevant directement le faisceau lumineux sortant de la chambre de mesure, le récepteur étant apte à délivrer un courant lorsqu'il reçoit un flux lumineux,
     - un système de commande (16) comprenant :

       - un système de pilotage (17) de l'émetteur de lumière configuré pour faire varier l'intensité lumineuse du faisceau lumineux émis entre une valeur minimale et une valeur maximale,
       - un système de mesure (18) du courant délivré par le récepteur de lumière comprenant :

         ∘ un convertisseur courant-tension (19) recevant le courant délivré par le récepteur (14) de lumière et délivrant une tension, ce convertisseur (19) comprenant un commutateur commandé (190) distribuant le courant dans un circuit choisi parmi au moins deux circuits d'impédance (191, 192) présentant des impédances différentes,

◦ un amplificateur à gain variable (20) recevant la tension délivrée par le convertisseur courant-tension (19) et délivrant une tension égale ou proportionnelle à la tension entrante,

◦ un convertisseur analogique-numérique (21) recevant la tension délivrée par l'amplificateur à gain variable (20) et délivrant un signal numérique représentatif de la quantité de courant délivrée par le récepteur de lumière,

- un système de gestion (22) du système de commande (16) de chaque cellule de mesure, configuré pour commander le système de pilotage (17) de l'émetteur de lumière, le commutateur (190) du convertisseur courant-tension (19) et l'amplificateur à gain variable (20) de chaque système de commande (16).

2. Dispositif de mesure (1) selon la revendication 1, **caractérisé en ce que** le système de pilotage (17) de l'émetteur de lumière est un système de pilotage de l'intensité du courant alimentant l'émetteur.

3. Dispositif de mesure (1) selon la revendication 1 ou 2, **caractérisé en ce que** l'amplificateur à gain variable (20) est intégré au convertisseur analogique-numérique (21).

4. Dispositif de mesure (1) selon l'une quelconque des revendications 1 à 3, **caractérisé en ce que** l'impédance de chaque circuit d'impédance du convertisseur courant-tension est choisie de sorte que, dans une plage d'intensités de courant, la tension délivrée par l'un des circuits d'impédance présente une plage d'amplitude recoupant la plage d'amplitude de la tension délivrée par un autre circuit d'impédance.

5. Dispositif de mesure (1) selon l'une quelconque des revendications 1 à 4, **caractérisé en ce que** chaque chambre de mesure (101) présente deux éléments optiques (102, 103) formant des parois opposées de la chambre de mesure, la distance minimale séparant les deux éléments optiques (102, 103) dans la direction d'émission présentant une valeur comprise dans la plage de 0,4 à 1,2mm, de préférence de 0,5 à 1mm.

6. Dispositif de mesure (1) selon l'une quelconque des revendications 1 à 5, **caractérisé en ce que** l'émetteur (12) et le récepteur (14) de chaque cellule de mesure (10) présentent respectivement une ouverture de sortie (120) du faisceau lumineux et une zone sensible (140), et **en ce que** ladite ouverture de sortie et ladite zone sensible sont chacune positionnées à l'intérieur d'un logement (205, 206) étanche aux rayonnements lumineux provenant de l'extérieur de la cellule de mesure, chaque logement débouchant uniquement sur la chambre de mesure (101), sur des parois opposées de celle-ci.

7. Dispositif de mesure (1) selon l'une quelconque des revendications 1 à 6, **caractérisé en ce que** chaque cellule de mesure (101) comprend une entrée (104) et une sortie (105) de fluide et **en ce que** le dispositif de mesure (1) comprend un circuit de fluide (106) associé à chaque chambre de mesure (101) et relié à la sortie (105) de fluide de celle-ci, le circuit de fluide (106) étant équipé d'un organe de mise en circulation du fluide (107).

8. Dispositif de mesure (1) selon la revendication 7, **caractérisé en ce que** chaque circuit de fluide (106) comprend un ou plusieurs des éléments suivants :

- au moins un réservoir (110) et au moins une conduite d'injection (109) de liquide reliée à chaque réservoir (110),
- une enceinte de mélange (113) présentant une entrée (114) et une sortie (115) connectées au circuit de fluide (106),
- au moins un organe de régulation de température (23, 24).

9. Dispositif de mesure (1) selon la revendication 7 ou 8, **caractérisé en ce que** le circuit de fluide (106) forme une boucle fermée à l'intérieur de laquelle circule le milieu.

10. Dispositif de mesure (1) selon l'une quelconque des revendications 7 à 9, **caractérisé en ce qu'**il comprend des moyens d'injection de liquide en continu à l'intérieur du circuit de fluide, notamment à l'intérieur de conduites du circuit de fluide.

11. Procédé de mesure du seuil de floculation d'un milieu colloïdal, notamment d'un milieu colloïdal contenant des asphaltènes, par addition de solvant aliphatique mis en œuvre par un dispositif de mesure (1) du seuil de floculation d'un milieu colloïdal selon l'une quelconque des revendications 1 à 10, comprenant les étapes suivantes :

(i) on introduit le milieu à l'intérieur de la chambre de mesure de la cellule de mesure fonctionnant par transmission optique directe du dispositif de mesure (1),

(i1) optionnellement, une étape de dilution dudit milieu avec une quantité prédéterminée de solvant aliphatique préalablement à l'étape (i),
(ii) à l'aide du système de gestion du dispositif de mesure du seuil de floculation, on règle une intensité lumineuse du faisceau lumineux émis par l'émetteur, on commande le commutateur du convertisseur courant-tension pour sélectionner un circuit d'impédance et on sélectionne un gain de l'amplificateur à gain variable de manière à obtenir un signal détectable par le convertisseur analogique-numérique,
(iii) on détermine le seuil de floculation à l'aide du dispositif de mesure après addition de la quantité de solvant aliphatique nécessaire à la floculation, optionnellement on modifie le gain de l'amplificateur à gain variable du dispositif de mesure en cours d'addition, optionnellement, le solvant aliphatique est ajouté en continu, et notamment à débit constant, et on procède aux mesures à l'aide du dispositif de mesure alors que le solvant aliphatique est en cours d'addition.

12. Procédé selon la revendication 11 dans lequel l'émetteur émet un faisceau lumineux dans le domaine du NIR et on détermine l'occurrence de la floculation par détermination du pic d'absorption.

13. Procédé selon l'une des revendications 11 à 12, dans lequel on détermine l'occurrence de la floculation à une température prédéterminée réglable.

14. Procédé selon l'une quelconque des revendications 11 à 13, dans lequel la lumière est constituée de longueurs d'ondes appartenant à un domaine spectral choisi parmi le domaine spectral du proche infrarouge et le domaine spectral de l'infra- rouge.

15. Procédé de détermination de la stabilité d'un mélange comprenant des asphaltènes par mise en œuvre au moins deux fois du procédé selon l'une des revendications 11 à 14 sur un milieu contenant le mélange et une quantité donnée de solvant aromatique, à des taux de dilution différents, optionnellement, le couple solvant aromatique/solvant aliphatique utilisé est le couple toluène/n- heptane.

**Patentansprüche**

1. Messvorrichtung (1) zum Messen der Flockungsschwelle eines kolloidalen Mediums durch Zugabe eines aliphatischen Lösungsmittels, wobei die Messvorrichtung Folgendes umfasst:

- mindestens eine Messzelle (10), die durch direkte optische Übertragung betrieben wird und eine Messkammer (101) aufweist, die dazu vorgesehen ist, das Medium zu empfangen, und, zugeordnet zu jeder Messzelle:

- einem Lichtsender (12), der konfiguriert ist, um ein in die Messkammer eintretendes Lichtbündel entsprechend einer Sendungsrichtung auszusenden,
- einem photoelektrischen Lichtempfänger (14), der das aus der Messkammer austretende Lichtbündel direkt empfängt, wobei der Empfänger geeignet ist, einen Strom zu liefern, wenn er einen Lichtstrom empfängt,
- ein Steuersystem (16), das Folgendes umfasst:

- ein Pilotsystem (17) des Lichtsenders, das konfiguriert ist, um die Lichtstärke des ausgesendeten Lichtbündels zwischen einem Minimalwert und einem Maximalwert zu variieren,
- ein Messsystem (18) für den vom Lichtempfänger gelieferten Strom, wobei das Messsystem Folgendes umfasst:

◦ einen Strom-Spannungs-Wandler (19), der den vom Lichtempfänger (14) gelieferten Strom empfängt und eine Spannung liefert, wobei dieser Wandler (19) einen gesteuerten Schalter (190) umfasst, der den Strom in eine Schaltung verteilt, die aus mindestens zwei unterschiedliche Impedanzen aufweisenden Impedanzschaltungen (191, 192) ausgewählt ist,
◦ einen Verstärker mit variabler Verstärkung (20), der die vom Strom-Spannungs-Wandler (19) gelieferte Spannung empfängt und eine Spannung liefert, die gleich oder proportional zur eintretenden Spannung ist,
◦ einen Analog-Digital-Wandler (21), der die vom Verstärker mit variabler Verstärkung (20) gelieferte Spannung empfängt und ein digitales Signal liefert, das die vom Lichtempfänger gelieferte Strommenge darstellt,

- ein Verwaltungssystem (22) des Steuersystems (16) jeder Messzelle, das konfiguriert ist, um das Pilotsystem (17) des Lichtsenders, den Schalter (190) des Strom-Spannungs-Wandlers (19) und den Verstärker mit variabler Verstärkung (20) jedes Steuersystems (16) zu steuern.

**2.** Messvorrichtung (1) nach Anspruch 1, **dadurch gekennzeichnet, dass** das Pilotsystem (17) des Lichtsenders ein Pilotsystem für die den Sender speisende Stromstärke ist.

**3.** Messvorrichtung (1) nach Anspruch 1 oder 2, **dadurch gekennzeichnet, dass** der Verstärker mit variabler Verstärkung (20) in den Analog-Digital-Wandler (21) integriert ist.

**4.** Messvorrichtung (1) nach einem beliebigen der Ansprüche 1 bis 3, **dadurch gekennzeichnet, dass** die Impedanz jeder Impedanzschaltung des Strom-Spannungs-Wandlers so ausgewählt ist, dass, in einem Bereich von Stromstärken, die von einer der Impedanzschaltungen gelieferte Spannung einen Amplitudenbereich aufweist, der den Amplitudenbereich der von einer anderen Impedanzschaltung gelieferten Spannung schneidet.

**5.** Messvorrichtung (1) nach einem beliebigen der Ansprüche 1 bis 4, **dadurch gekennzeichnet, dass** jede Messkammer (101) zwei optische Elemente (102, 103) aufweist, die gegenüberliegende Wände der Messkammer bilden, wobei der die beiden optischen Elemente (102, 103) in Sendungsrichtung trennende Mindestabstand einen Wert innerhalb des Bereichs von 0,4 bis 1,2 mm, vorzugsweise von 0,5 bis 1 mm aufweist.

**6.** Messvorrichtung (1) nach einem beliebigen der Ansprüche 1 bis 5, **dadurch gekennzeichnet, dass** der Sender (12) und der Empfänger (14) jeder Messzelle (10) jeweils eine Ausgangsöffnung (120) für das Lichtbündel und eine empfindliche Zone (140) aufweisen, und dass die Ausgangsöffnung und die empfindliche Zone jeweils im Inneren eines Gehäuses (205, 206) positioniert sind, das undurchlässig für von außerhalb der Messzelle kommende Lichtstrahlen ist, wobei jedes Gehäuse nur an gegenüberliegenden Wänden der Messkammer (101) in diese mündet.

**7.** Messvorrichtung (1) nach einem beliebigen der Ansprüche 1 bis 6, **dadurch gekennzeichnet, dass** jede Messzelle (101) einen Eingang (104) und einen Ausgang (105) für Fluid umfasst und dass die Messvorrichtung (1) eine jeder Messkammer (101) zugeordnete und mit deren Fluidausgang (105) verbundene Fluidschaltung (106) umfasst, wobei die Fluidschaltung (106) mit einem Organ (107) zum Umwälzen des Fluids ausgestattet ist.

**8.** Messvorrichtung (1) nach Anspruch 7, **dadurch gekennzeichnet, dass** jede Fluidschaltung (106) eines oder mehrere der folgenden Elemente umfasst:

- mindestens einen Behälter (110) und mindestens eine mit jedem Behälter (110) verbundene Einspritzleitung (109) für Flüssigkeit,
- ein Mischgefäß (113), das einen Eingang (114) und einen Ausgang (115) aufweist, die an die Fluidschaltung (106) angeschlossen sind,
- mindestens ein Organ zur Regulierung der Temperatur (23, 24).

**9.** Messvorrichtung (1) nach Anspruch 7 oder 8, **dadurch gekennzeichnet, dass** die Fluidschaltung (106) eine geschlossene Schleife bildet, in deren Innerem sich das Medium umwälzt.

**10.** Messvorrichtung (1) nach einem beliebigen der Ansprüche 7 bis 9, **dadurch gekennzeichnet, dass** sie Mittel zur kontinuierlichen Einspritzung von Flüssigkeit in das Innere der Fluidschaltung, insbesondere in das Innere von Leitungen der Fluidschaltung, umfasst.

**11.** Verfahren zur Messung der Flockungsschwelle eines kolloidalen Mediums, insbesondere eines Asphaltene enthaltenden kolloidalen Mediums, durch Zugabe eines aliphatischen Lösungsmittels, das durch eine Messvorrichtung (1) zum Messen der Flockungsschwelle eines kolloidalen Mediums nach einem beliebigen der Ansprüche 1 bis 10 durchgeführt wird und die folgenden Schritte umfasst:

(i) Einführen des Mediums in das Innere der Messkammer der Messzelle, die durch direkte optische Übertragung der Messvorrichtung (1) betrieben wird,
(i1) optional einen Schritt zum Verdünnen des Mediums mit einer vorbestimmten Menge eines aliphatischen Lösungsmittels vor dem Schritt (i),
(ii) mit Hilfe des Verwaltungssystems der Messvorrichtung zum Messen der Flockungsschwelle, Regeln einer Lichtstärke des vom Sender ausgesendeten Lichtbündels, Steuern des Schalters des Strom-Spannungs-Wandlers zum Selektieren einer Impedanzschaltung und Selektieren einer Verstärkung des Verstärkers mit variabler Verstärkung, sodass vom Analog-Digital-Wandler ein erfassbares Signal erhalten wird,
(iii) Bestimmen der Flockungsschwelle mit Hilfe der Messvorrichtung nach Zugabe der zur Flockung erforderlichen Menge an aliphatischem Lösungsmittel, optional Modifizieren der Verstärkung des Verstärkers mit variabler Verstärkung der Messvorrichtung im Laufe der Zugabe, optional wird das aliphatische Lösungsmittel kontinuierlich beigemengt, und insbesondere mit konstanter Durchflussmenge, und Durchführen der Messungen mit Hilfe der Messvorrichtung bei laufender Zugabe des aliphatischen Lösungsmittels.

**12.** Verfahren nach Anspruch 11, wobei der Sender ein Lichtbündel im NIR-Spektrum aussendet und das Auftreten der Flockung durch Bestimmen des Absorptionspeaks bestimmt wird.

**13.** Verfahren nach einem der Ansprüche 11 bis 12, wobei das Bestimmen des Auftretens der Flockung bei einer vorbestimmten, regelbaren Temperatur erfolgt.

**14.** Verfahren nach einem beliebigen der Ansprüche 11 bis 13, wobei das Licht aus Wellenlängen besteht, die zu einem Spektralbereich gehören, der aus dem Spektralbereich des nahen Infrarots und dem Spektralbereich des Infrarots ausgewählt ist.

**15.** Verfahren zur Bestimmung der Stabilität einer Mischung, die Asphaltene umfasst, durch mindestens zweimalige Durchführung des Verfahrens nach einem der Ansprüche 11 bis 14 an einem die Mischung und eine bestimmte Menge eines aromatischen Lösungsmittels enthaltenden Medium bei unterschiedlichen Verdünnungsgraden, wobei optional das verwendete Paar aromatisches Lösungsmittel/aliphatisches Lösungsmittel das Paar Toluol/n-Heptan ist.

**Claims**

**1.** A measurement device (1) for measuring the threshold for flocculation of a colloidal medium by addition of aliphatic solvent, comprising:

- at least one measurement cell (10) operating by direct optical transmission and having a measurement chamber (101) intended to receive the medium, and, associated with each measurement cell:

- an emitter (12) of light configured to emit a light beam entering into the measurement chamber along a direction of emission,
- a receiver (14) of photoelectric light directly receiving the light beam leaving the measurement chamber, the receiver being suitable for delivering a current when the receiver receives a light flux,
- a control system (16) comprising:

- a piloting system (17) for the light emitter configured for varying the light intensity of the light beam emitted between a minimum value and a maximum value,
- a measurement system (18) of the current delivered by the light receiver comprising:

○ a current-to-voltage converter (19) receiving the current delivered by the receiver (14) of the light and delivering a voltage, said converter (19) comprising a controlled switch (190) distributing the current in a circuit chosen amongst at least two impedance circuits (191, 192) having different impedances,
○ a variable gain amplifier (20) receiving the voltage delivered by the current-to-voltage converter (19) and delivering a voltage equal or proportional to the input voltage,
○ an analog-to-digital converter (21) receiving the voltage delivered by the variable gain amplifier (20) and delivering a digital signal representative of the amount of current delivered by the light receiver,

- a management system (22) of the control system (16) of each measurement cell, configured for controlling the piloting system (17) of the light emitter, the switch (190) of the current-to-voltage converter (19) and the variable gain amplifier (20) of each control system (16).

**2.** The measurement device (1) according to claim 1, **characterized in that** the piloting system (17) of the light emitter is a current intensity piloting system supplying the emitter.

**3.** The measurement device (1) according to claim 1 or 2, **characterized in that** the variable gain amplifier (20) is integrated into the analog-to-digital converter (21).

**4.** The measurement device (1) according to any of claims 1 to 3, **characterized in that** the impedance of each impedance circuit of the current-to-voltage converter is chosen such that, over a range of current intensities, the voltage delivered by one of the impedance circuits has a range of amplitude overlapping the range of amplitude of the voltage delivered by another impedance circuit.

**5.** The measurement device (1) according to any of claims 1 to 4, **characterized in that** each measurement chamber (101) has two optical elements (102, 103) forming opposite walls of the measurement chamber, the minimum distance separating the two optical elements (102, 103) in the direction of emission having a value comprises within a range from 0.4 to 1.2mm, preferably 0.5 to 1 mm.

**6.** The measurement device (1) according to any of claims 1 to 5, **characterized in that** the emitter (12) and the receiver (14) of each measurement cell (10) has an output opening (120) for the light beam and a sensitive zone (140) respectively and **in that** said output opening and said sensitive zone are each positioned in the interior of a housing (205, 206) light-tight to light radiation coming from outside the measurement cell, each housing opening only onto the measurement chamber (101), onto the opposite walls of the same.

7. The measurement device (1) according to any of claims 1 to 6, **characterized in that** each measurement cell (101) comprises a fluid inlet (104) and a fluid outlet (105) and **in that** the measurement device (1) comprises a fluid circuit (106) associated with each measurement chamber (101) and connected to the fluid outlet (105) of the same, the fluid circuit (106) being equipped with a fluid circulating member (107).

8. The measurement device (1) according to claim 7, **characterized in that** each fluid circuit (106) comprises one or a plurality of the following elements:

    - at least one tank (110) and at least one liquid injection pipe (109) connected to each tank (110),
    - a mixing chamber (113) having an inlet (114) and an outlet (115) connected to the fluid circuit (106),
    - at least one temperature regulation member (23, 24).

9. The measurement device (1) according to claim 7 or 8, **characterized in that** the fluid circuit (106) forms a closed loop inside of which the medium circulates.

10. The measurement device (1) according to any of claims 7 to 9, **characterized in that** said measurement device comprises means of continuous injection of a liquid into the interior of the fluid circuit, in particular into the interior of the fluid circuit pipes.

11. A method of measuring the threshold for flocculation of a colloidal medium, in particular of a colloidal medium containing asphaltenes, by addition of aliphatic solvent used by a measurement device (1) for measuring the threshold for flocculation of a colloidal medium according to any of claims 1 to 10, comprising the following steps:

    (i) the medium is introduced into the interior of the measurement chamber of the measurement cell operating by direct optical transmission of the measurement device (1),
    (i1) optionally, a step of dilution of said medium with a predetermined amount of aliphatic solvent prior to step (i),
(ii) using the management system of the measurement device for measuring the threshold for flocculation, the light intensity of the light beam emitted by the emitter is adjusted, the switch of the current-to-voltage converter is controlled for selecting an impedance circuit and a gain of a variable gain amplifier is selected so as to obtain a signal, detectable by the analog-to-digital converter,
(iii) the threshold for flocculation is determined using the measurement device after the addition of the amount of aliphatic solvent necessary for flocculation, optionally the gain of the variable gain amplifier of the measurement device is modified during the addition, optionally, the aliphatic solvent is continuously added and in particular at constant flow rate, and measurements are made using the measurement device while the aliphatic solvent is being added.

12. The method according to claim 11, wherein the emitter emits a light beam in the NIR domain and the occurrence of flocculation is determined by the determination of the absorption peak.

13. The method according to one of the claims 11 to 12, wherein the occurrence of flocculation is determined at an adjustable predetermined temperature.

14. The method according to any one of claims 11 to 13, wherein the light is composed of wavelengths belonging to a spectral domain chosen amongst the near infra-red spectral domain and the infra-red spectral domain.

15. A method for determining the stability of a mixture comprising asphaltenes by implementing at least twice the method according to one of the claims 11 to 14 on a medium containing the mixture and a given amount of aromatic solvent, at different dilution rates, optionally, the aromatic solvent/aliphatic solvent pair used is the toluene/n-heptane pair.

## COURBE DE PRECIPITATION D'UN PRODUIT NOIR

FR

0,6

0,5    1-Sa=FRmax

0,4    P1    Valeurs déduites

0,3

0,2    P2

0,1    Valeurs mesurées    1/(S-1)=Xmin

0    0    0,02    0,04    0,06    0,08    0,1    0,12

**1 / DILUTION**

Fig. 1

Figure 4

Fig. 2

Fig. 3

Fig. 5

Fig. 6a

Fig. 6b

Fig. 6c

Fig. 7

## RÉFÉRENCES CITÉES DANS LA DESCRIPTION

**Documents brevets cités dans la description**

- FR 2596522 A
- US 4628204 A
- FR 2655909

- US 10422782 B2
- WO 2005003754 A2
- EP 1751518 B1

**Littérature non-brevet citée dans la description**

- Flocculation Onset Titration of Petroleum Asphaltènes. *Energy & Fuels*, 1999, vol. 13, 315-322